# EUROPEAN PATENT SPECIFICATION

(11) **EP 2 879 497 B1**
(45) Date of publication and mention of the grant of the patent: **05.10.2022**
(21) Application number: 13745385.8
(22) Date of filing: 31.07.2013
(51) Int. Cl.: A01N 43/56, A01N 43/36, A01N 25/00, A01P 3/00

(54) **METHOD OF COMBATTING SOYBEAN SUDDEN DEATH SYNDROME USING SUBSTITUTED PYRAZOLAMIDES**
VERFAHREN ZUR BEKÄMPFUNG VON PLÖTZLICHEM SOJABOHNEN STERBEN MIT SUBSTITUIERTEN PYRAZOLAMIDEN
PROCEDE POUR COMBATTRE LE SYNDROME DE MORT DE GRAINES DE SOJA AVEC PYRAZOLAMIDES SUBSTITUES

(30) Priority: 06.08.2012 US 201261680031 P
(43) Date of publication of application: 10.06.2015
(73) Proprietor: Syngenta Participations AG, 4058 Basel (CH)
(72) Inventor: OLAYA-HUERTAS, Gilberto, Vero Beach, FL 32967 (US); SWART, Gina, Mercia, CH-4058 Basel (CH)
(74) Representative: SYNGENTA IP
(86) International application number: PCT/EP2013/066082
(87) International publication number: WO 2014/023628

(56) References cited:
- WO-A1-2010/000612
- WO-A1-2010/084078
- WO-A1-2010/135324
- WO-A1-2011/048120
- WO-A1-2011/163474
- WO-A1-2012/041874
- WO-A1-2012/099875
- WO-A2-2010/063700

## Description

The present invention relates to a method for reducing the occurrence of Sudden Death Syndrome in crops of useful plants, especially soybeans, which method comprising the application of a pyrazolyl-carboxamide to the plant, seed and/or soil.

Sudden Death Syndrome (SDS) is a fungal disease, especially of soybeans, found in the North America caused by *Fusarium virguliforme (Fusarium solani* f.sp. *glycines).* Likewise, *Fusarium brasiliense* sp. nov., *Fusarium cuneirostrum* sp. nov. and *Fusarium tucumaniae* are known precursors to SDS on other global regions, for example South America. *Fusarium virguliforme* produces toxins that are translocated from the tap root to the foliage. Infected plants will begin to show distinct yellow speckling or spotting between the veins (interveinal chlorosis). As the disease progresses these areas become larger and in severe cases the entire area between the veins becomes brown and dry. The disease is of major concern because of its potential ability to reduce yields, from a slight yield loss to 100%, depending on the soybean variety. The pathogen has been isolated from soybean roots and lower stems, but not from leaves. Therefore, combatting this disease by applying a fungicide to the leaves is not possible.

Solutions of this problem are presently rare. WO 2012/071520 describes a method for reducing the occurrence of Sudden Death Syndrome, comprising applying a pyridinyl ethylbenzamide to plant seed, soil or roots. WO 2011/163474 describes a method for reducing the occurrence of Sudden Death Syndrome, comprising applying thiabendazole to plant seed.

There is therefore proposed in accordance with the present invention a method according to claim 1.

The compound of formula I can occur in two enantiomeric forms of formula la and lb:

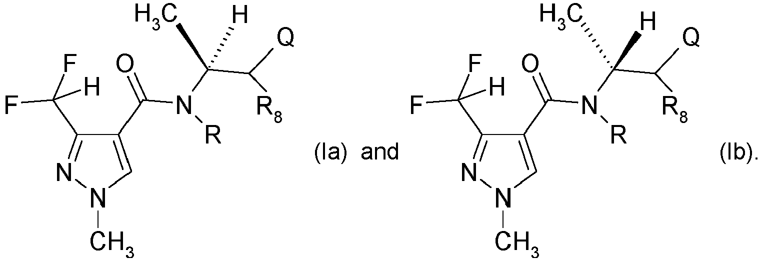

The compound used in the invention encompasses both enantiomeric forms of the compound of formula I, and its preparation is described in WO 2010/063700, WO 2010/084078 and WO 2008/151828.

A preferred embodiment of the invention comprises a method according to claim 1.

Another preferred embodiment of the invention is a method according to claim 2.

A further preferred embodiment of the invention is a method according to claim 3.

The plant seed is soybean seed.

Also disclosed is a soybean seed treated with a composition comprising a compound of formula I in an amount for reducing the occurrence of phytopathogenic fungi selected from the group consisting of *Fusarium virguliforme, Fusarium brasiliense* sp. nov., *Fusarium cuneirostrum* sp. nov. and *Fusarium tucumaniae* in plants.

The compounds 3-difluoromethyl-1-methyl-1H-pyrazole-4-carboxylic acid [2-(2,4-dichlorophenyl)-2-methoxy-1-methyl-ethyl]-amide and 3-difluoromethyl-1-methyl-1H-pyrazole-4-carboxylic acid methoxy-[1-methyl-2-(2,4,5-trichlorothiophene-3-yl)ethyl]amide are especially useful for the methods disclosed herein.

The term "plants" or "useful plants" is to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®} , Herculex I^{®} and LibertyLink^{®}.

The term "useful plants" is to be understood as including also useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CrylA(b) toxin); YieldGard Rootworm^{®} (maize variety that expresses a CηIIIB(b1) toxin); YieldGard Plus^{®} (maize variety that expresses a CrylA(b) and a CηIIIB(b1) toxin); Starlink^{®} (maize variety that expresses a Cry9(c) toxin); Herculex I^{®} (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CrylA(c) toxin); Bollgard I^{®} (cotton variety that expresses a CrylA(c) toxin); Bollgard II^{®} (cotton variety that expresses a CrylA(c) and a CryIIA(b) toxin); VIPCOT^{®} (cotton variety that expresses a VIP toxin); NewLeaf^{®} (potato variety that expresses a CryIIIA toxin); NatureGard^{®} Agrisure^{®} GT Advantage (GA21 glyphosate-tolerant trait), Agrisure^{®}CB Advantage (Bt11 corn borer (CB) trait), Agrisure^{®}RW (corn rootworm trait) and Protecta^{®}.

The term "plants" or "useful plants" is to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

According to the invention "plants" and "useful plants" typically comprise the following species of plants: grape vines; cereals, such as wheat, barley, rye or oats; beet, such as sugar beet or fodder beet; fruits, such as pomes, stone fruits or soft fruits, for example apples, pears, plums, peaches, almonds, cherries, strawberries, raspberries or blackberries; leguminous plants, such as beans, lentils, peas or soybeans; oil plants, such as rape, mustard, poppy, olives, sunflowers, coconut, castor oil plants, cocoa beans or groundnuts; cucumber plants, such as marrows, cucumbers or melons; fibre plants, such as cotton, flax, hemp or jute; citrus fruit, such as oranges, lemons, grapefruit or mandarins; vegetables, such as spinach, lettuce, asparagus, cabbages, carrots, onions, tomatoes, potatoes, cucurbits or paprika; lauraceae, such as avocados, cinnamon or camphor; maize; tobacco; nuts; coffee; sugar cane; tea; vines; hops; durian; bananas; natural rubber plants; turf or ornamentals, such as flowers, shrubs, broad-leaved trees or evergreens, for example conifers. This list does not represent any limitation.

The compound of formula I can be used in unmodified form or, preferably, together with carriers and adjuvants conventionally employed in the art of formulation.

To this end compound of formula I and inert carriers are conveniently formulated in known manner to emulsifiable concentrates, coatable pastes, directly sprayable or dilutable solutions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compound of formula I or compositions, comprising the compound of formula I as acitve ingredient and an inert carrier, can be applied to the locus of the plant or plant to be treated, simultaneously or in succession with further compounds. These further compounds can be e.g. fertilizers or micronutrient donors or other preparations which influence the growth of plants.

The frequency of application and the rate of application will depend on the risk of infestation by the corresponding pathogen. The compound of formula I can penetrate the plant through the roots via the soil (systemic action) by drenching the locus of the plant with a liquid formulation, or by applying the compounds in solid form to the soil, e.g. in granular form (soil application). The compound of formula I may also be applied to seeds (coating) by impregnating the seeds or tubers either with a liquid formulation of the fungicide or coating them with a solid formulation.

A formulation, i.e. a composition comprising the compound of formula I and, if desired, a solid or liquid adjuvant, is prepared in a known manner, typically by intimately mixing and/or grinding the compound with extenders, for example solvents, solid carriers and, optionally, surface-active compounds (surfactants).

The agrochemical formulations will usually contain from 0.1 to 99% by weight, preferably from 0.1 to 95% by weight, of the compound of formula I, 99.9 to 1% by weight, preferably 99.8 to 5% by weight, of a solid or liquid adjuvant, and from 0 to 25% by weight, preferably from 0.1 to 25% by weight, of a surfactant.

Whereas it is preferred to formulate commercial products as concentrates, the end user will normally use dilute formulations.

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient rates of application are from 10mg to 1g of active substance per kg of seeds. The rate of application for the desired action can be determined by experiments. It depends for example on the type of action, the developmental stage of the useful plant, and on the the application (location, timing, application method) and can, owing to these parameters, vary within wide limits.

Formulation examples for the compound of formula I:

### Example F-1.1 to F-1.2: Emulsifiable concentrates

| Components | F-1.1 F-1.2 | |
|---|---|---|
| compound I | 25% | 50% |
| calcium dodecylbenzenesulfonate | 5% | 6% |
| castor oil polyethylene glycol ether | | |
| (36 mol ethylenoxy units) | 5% | - |
| tributylphenolpolyethylene glycol ether | | |
| (30 mol ethylenoxy units) | - | 4% |
| cyclohexanone | - | 20% |
| xylene mixture | 65% | 20% |

Emulsions of any desired concentration can be prepared by diluting such concentrates with water.

### Example F-2: Emulsifiable concentrate

| Components | F-2 |
|---|---|
| compound I | 10% |
| octylphenolpolyethylene glycol ether | |
| (4 to 5 mol ethylenoxy units) | 3% |
| calcium dodecylbenzenesulfonate | 3% |
| castor oil polyglycol ether | |
| (36 mol ethylenoxy units) | 4% |
| cyclohexanone | 30% |
| xylene mixture | 50% |

Emulsions of any desired concentration can be prepared by diluting such concentrates with water.

### Examples F-3.1 to F-3.4: Solutions

| Components | | F-3.1 | F-3.2 | F-3.3 | F-3.4 |
|---|---|---|---|---|---|
| compound I | | | | | |
| | 80% | 10% | 5% | 95% | |
| propylene glycol monomethyl ether | | 20% | - | - | - |
| polyethylene glycol (relative molecular | | | | | |
| mass: 400 atomic mass units) | | - | 70% | - | - |
| N-methylpyrrolid-2-one | | - | 20% | - | - |
| epoxidised coconut oil | | - | - | 1% | 5% |
| benzin (boiling range: 160-190°) | | - | - | 94% | - |
| The solutions are suitable for use in the form of microdrops. | | | | | |
| Examples F-4.1 to F-4.4: Granulates | | | | | |

| Components | | F-4.1 | F-4.2 | F-4.3 | F-4.4 |
|---|---|---|---|---|---|
| compound I | | | | | |
| | 5% | 10% | 8% | 21% | |
| kaolin | | 94% | - | 79% | 54% |
| highly dispersed silicic acid | | 1% | - | 13% | 7% |
| attapulgite | | - | 90% | - | 18% |

The novel compound is dissolved in dichloromethane, the solution is sprayed onto the carrier and the solvent is then removed by distillation under vacuum.

### Examples F-5.1 and F-5.2: Dusts

| Components | F-5.1 | F-5.2 | | |
|---|---|---|---|---|
| compound I | | 2% | | 5% |
| highly dispersed silicic acid | 1% | 5% | | |
| talcum | 97% | - | | |
| kaolin | - | 90% | | |

Ready for use dusts are obtained by intimately mixing all components.

### Examples F-6.1 to F-6.3: Wettable powders

| Components | F-6.1 | F-6.2 | F-6.3 |
|---|---|---|---|
| compound I | 25% | 50% | 75% |
| sodium lignin sulfonate | 5% | 5% | - |
| sodium lauryl sulfate | 3% | - | 5% |
| sodium diisobutylnaphthalene sulfonate | - | 6% | 10% |
| octylphenolpolyethylene glycol ether | | | |
| (7 to 8 mol ethylenoxy units) | - | 2% | - |
| highly dispersed silicic acid | 5% | 10% | 10% |
| kaolin | 62% | 27% | - |

All components are mixed and the mixture is thoroughly ground in a suitable mill to give wettable powders which can be diluted with water to suspensions of any desired concentration.

### Example F7: Flowable concentrate for seed treatment

| | |
|---|---|
| compound I | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

The methods according to the invention can also be used in combination with other additional pesticides in order to increase the pest control spectrum.

Said combination can contain components (A) and (B), wherein component (A) is the compound of formula I and component (B) is a compound selected from the group consisting of
(B1) a strobilurin fungicide,
(B2) an azole fungicide,
(B3) a morpholine fungicide,
(B4) an anilinopyrimidine fungicide,
(B5) a fungicide selected from the group consisting of
   anilazine, arsenates, benalaxyl, benalaxyl-M, benodanil, benomyl, benthiavalicarb, benthiavalicarb-isopropyl, biphenyl, bitertanol, blasticidin-S, bordeaux mixture, boscalid, bupirimate, cadmium chloride, captafol, captan, carbendazim, carbon disulfide, carboxin, carpropamid, cedar leaf oil, chinomethionat, chlorine, chloroneb, chlorothalonil, chlozolinate, cinnamaldehyde, copper, copper ammoniumcarbonate, copper hydroxide, copper octanoate, copper oleate, copper sulphate, cyazofamid, cycloheximide, cymoxanil, dichlofluanid, dichlone, dichloropropene, diclocymet, diclomezine, dicloran, diethofencarb, diflumetorim, dimethirimol, dimethomorph, dinocap, dithianon, dodine, edifenphos, ethaboxam, ethirimol, etridiazole, famoxadone, fenamidone, fenaminosulf, fenamiphos, fenarimol, fenfuram, fenhexamid, fenoxanil, fenpiclonil, fentin acetate, fentin chloride, fentin hydroxide, ferbam, ferimzone, fluazinam, fludioxonil, flusulfamide, flusulfamide, flutolanil, folpet, formaldehyde, fosetyl-aluminium, fthalide, fuberidazole, furalaxyl, furametpyr, flyodin, fuazatine, hexachlorobenzene, hymexazole, iminoctadine, iodocarb, iprobenfos, iprodione, iprovalicarb, isoprothiolane, kasugamycin, mancozeb, maneb, manganous dimethyldithiocarbamate, mefenoxam, mepronil, mercuric chloride, mercury, metalaxyl, methasulfocarb, metiram, metrafenone, nabam, neem oil (hydrophobic extract), nuarimol, octhilinone, ofurace, oxadixyl, oxine copper, oxolinic acid, oxycarboxin, oxytetracycline, paclobutrazole, paraffin oil, paraformaldehyde, pencycuron, pentachloronitrobenzene, pentachlorophenol, penthiopyrad, perfurazoate, phosphoric acid, polyoxin, polyoxin D zinc salt, potassium bicarbonate, probenazole, procymidone, propamocarb, propineb, proquinazid, prothiocarb, pyrazophos, pyrifenox, pyroquilon, quinoxyfen, quintozene, silthiofam, sodium bicarbonate, sodium diacetate, sodium propionate, streptomycin, sulphur, TCMTB, tecloftalam, tecnazene, thiabendazole, thifluzamide, thiophanate, thiophanate-methyl, thiram, tolclofos-methyl, tolyfluanid, triazoxide, trichoderma harzianum, tricyclazole, triforine, triphenyltin hydroxide, validamycin, vinclozolin, zineb, ziram, zoxamide, 1,1-bis(4-chlorophenyl)-2-ethoxyethanol, 2,4-dichlorophenyl benzenesulfonate, 2-fluoro-*N*-methyl-*N*-1-naphthylacetamide, 4-chlorophenyl phenyl sulfone,
   a compound of formula B-5.1 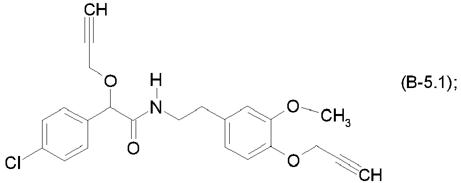
   a compound of formula B-5.2 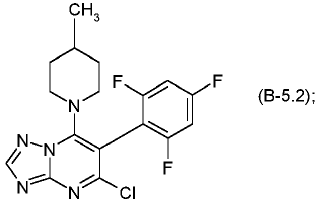
   a compound of formula B-5.3 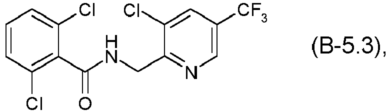
   a compound of formula B-5.4 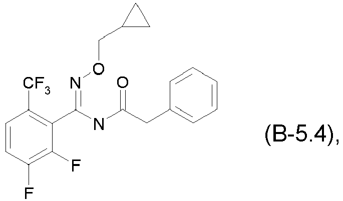
   a compound of formula B-5.5 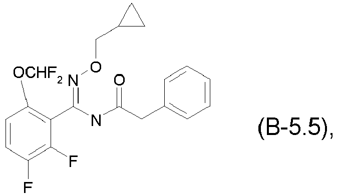
   a compound of formula B-5.6 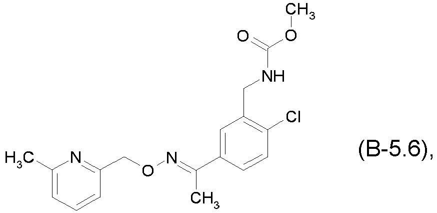
   a compound of formula B-5.7 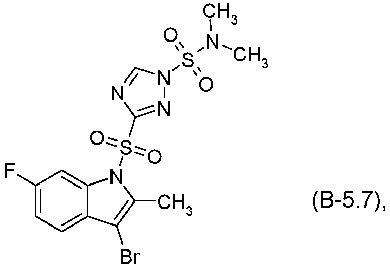
   3-difluoromethyl-1-methyl-1H-pyrazole-4-carboxylic acid (2-bicyclopropyl-2-yl-phenyl)-amide (compound B-5.8), 3-difluoromethyl-1-methyl-1H-pyrazole-4-carboxylic acid (9-isopropyp-1,2,3,4-tetrahydro-1,4-methano-naphthalen-5-yl)-amide (compound B-5.9), 1,3-dimethyl-5-fluoro-1H-pyrazole-4-carboxylic acid [2-(1,3-dimethylbutyl)phenyl]-amide (compound B-5.10), 3-difluoromethyl-1-methyl-1H-pyrazole-4-carboxylic acid (3',4'-dichloro-5-fluoro-1,1'-biphenyl-2-yl)-amide (compound B-5.11), N-{2-[3-chloro-5-(trifluoromethyl)pyridin-2-yl]ethyl}-2-(trifluoromethyl)benzamid (compound B-5.12), 3-difluoromethyl-1-methyl-1H-pyrazole-4-carboxylic acid N-[2-(1,1,2,2-tetrafluoroethoxy)phenyl]-amide (compound B-5.13), 3-difluoromethyl-1-methyl-1H-pyrazole-4-carboxylic acid N-[2-(1,1,2,3,3,3-hexafluoropropoxy)phenyl]-amide (compound B-5.14), 3-difluoromethyl-1-methyl-1H-pyrazole-4-carboxylic acid N-[2-(2-chloro-1,1,2-trifluoroethoxy)phenyl]-amide (compound B-5.15), 3-difluoromethyl-1-methyl-1H-pyrazole-4-carboxylic acid N-(4'-trifluoromethyl-biphen-2-yl)-amide (compound B-5.16), 3-difluoromethyl-1-methyl-1H-pyrazole-4-carboxylic acid N-(2'-trifluoromethyl-biphen-2-yl)-amide (compound B-5.17) and 3-difluoromethyl-1-methyl-1H-pyrazole-4-carboxylic acid N-(2'-trifluoromethyl-biphen-2-yl)-amide (compound B-5.18);
(B6) a plant-bioregulator selected from the group consisting of
   acibenzolar-S-methyl, chlormequat chloride, ethephon, mepiquat chloride and trinexapac-ethyl;
(B7) an insecticide selected from the group consisting of
   abamectin, clothianidin, emamectin benzoate, imidacloprid, tefluthrin, thiamethoxam,
   a compound of formula B-7.1 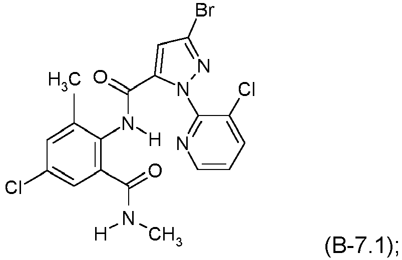 and
   a compound of formula B-7.2; 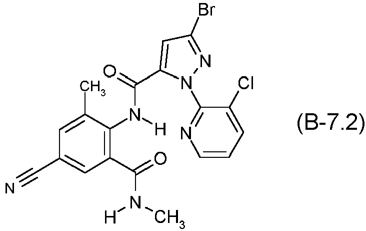 ; a
   a compound of formula B-7.3 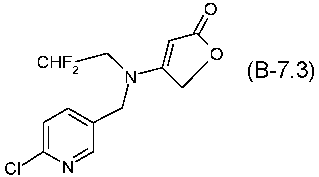 and
(B8) glyphosate, a compound of formula V 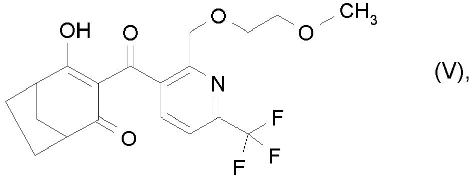 fomesafen, and
(B9) isopyrazam, sedaxane, a compound of formula (VI) 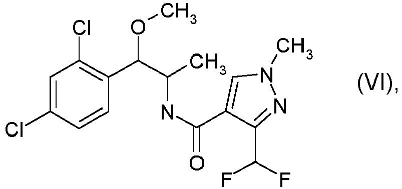 a compound of formula (VII) 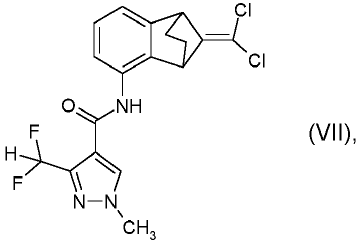 1-[4-[4-[(5S)5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone, 1-[4-[4-[5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone [1003318-67-9], and 3-difluoromethyl-1-methyl-1H-pyrazole-4-carboxylic acid (3',4',5'-trifluoro-biphenyl-2-yl)-amide.

As disclosed herein, preferred salts of glyphosate are the potassium, isopropylammonium, sodium, trimesium, ammonium and diammonium salts. Preferred salts of glufosinate are disclosed in US-A-4,168,963, a preferred salt is the ammonium salt.

As disclosed herein, a "racemic compound" means a mixture of at least two enantiomers in a ratio of substantially 50 : 50.

In general, the weight ratio of component (A) to component (B) is from 2000 : 1 to 1 : 1000. A non-limiting example for such weight ratios is compound of formula I : compound of formula B-2 is 10:1. The weight ratio of component (A) to component (B) is preferably from 100 : 1 to 1 : 100; more preferably from 20 : 1 to 1 : 50.

The active ingredient mixture of component (A) to component (B) comprises the compound of formula I and a further, other biocidally active ingredient or composition preferably in a mixing ratio of from 1000:1 to 1:1000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are understood to include, on the one hand, ratios by weight and also, on other hand, molar ratios.

It has been found, surprisingly, that certain weight ratios of component (A) to component (B) are able to give rise to synergistic activity. This synergistic activity is apparent from the fact that the fungicidal activity of the composition comprising component (A) and component (B) is greater than the sum of the fungicidal activities of component (A) and of component (B). This synergistic activity extends the range of action of component (A) and component (B) in two ways. Firstly, the rates of application of component (A) and component (B) are lowered whilst the action remains equally good, meaning that the active ingredient mixture still achieves a high degree of phytopathogen control even where the two individual components have become totally ineffective in such a low application rate range. Secondly, there is a substantial broadening of the spectrum of phytopathogens that can be controlled.

However, besides the actual synergistic action with respect to fungicidal activity, thecompositions disclosed herein can also have further surprising advantageous properties. Examples of such advantageous properties that may be mentioned are: more advantageuos degradability; improved toxicological and/or ecotoxicological behaviour; or improved characteristics of the useful plants including: emergence, crop yields, more developed root system, tillering increase, increase in plant height, bigger leaf blade, less dead basal leaves, stronger tillers, greener leaf colour, less fertilizers needed, less seeds needed, more productive tillers, earlier flowering, early grain maturity, less plant verse (lodging), increased shoot growth, improved plant vigor, and early germination.

The components (B) are known. Where the components (B) are included in "The Pesticide Manual" [The Pesticide Manual - A World Compendium; Thirteenth Edition; Editor: C. D. S. Tomlin; The British Crop Protection Council], they are described therein under the entry number given in round brackets hereinabove for the particular component (B); for example, the compound "abamectin" is described under entry number (1). Most of the components (B) are referred to hereinabove by a so-called "common name", the relevant "ISO common name" or another "common name" being used in individual cases. If the designation is not a "common name", the nature of the designation used instead is given in round brackets for the particular component (B); in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed.

The following components (B) are registered under a CAS-Reg. No.
aldimorph (CAS 91315-15-0); arsenates (CAS 1327-53-3); benalaxyl -M (CAS 98243-83-5); benthiavalicarb (CAS 413615-35-7); cadmium chloride (CAS 10108-64-2); cedar leaf oil (CAS 8007-20-3); chlorine (CAS 7782-50-5); cinnamaldehyde (CAS: 104-55-2); copper ammoniumcarbonate (CAS 33113-08-5); copper oleate (CAS 1120-44-1); iodocarb (3-lodo-2-propynyl butyl carbamate) (CAS 55406-53-6); hymexazole (CAS 10004-44-1); manganous dimethyldithiocarbamate (CAS 15339-36-3); mercury (CAS 7487-94-7; 21908-53-2; 7546-30-7); metrafenone (CAS 220899-03-6); neem oil (hydrophobic extract) (CAS 8002-65-1); orysastrobin CAS 248593-16-0); paraformaldehyde (CAS 30525-89-4); penthiopyrad (CAS 183675-82-3); phosphoric acid (CAS 7664-38-2); potassium bicarbonate (CAS 298-14-6); sodium bicarbonate (CAS 144-55-8); sodium diacetate (CAS 127-09-3); sodium propionate (CAS 137-40-6);TCMTB (CAS 21564-17-0); and tolyfluanid (CAS 731-27-1).

Compound B-1.1 ("enestrobin") is described in EP-0-936-213; compound B-3.1 ("flumorph") in US-6,020,332, CN-1-167-568, CN-1-155-977 and in EP-0-860-438; compound B-5.1 ("mandipropamid") in WO 01/87822; compound B-5.2 in WO 98/46607; compound B-5.3 ("fluopicolide") in WO 99/42447; compound B-5.4 ("cyflufenamid") in WO 96/19442; compound B-5.5 in WO 99/14187; compound B-5.6 ("pyribencarb") is registered under CAS-Reg. No. 325156-49-8; compound B-5.7 ("amisulbrom" or "ambromdole") is registered under CAS-Reg. No. 348635-87-0; compound B-5.8 (3-difluoromethyl-1-methyl-1H-pyrazole-4-carboxylic acid (2-bicyclopropyl-2-yl-phenyl)-amide) is described in WO 03/74491; compound B-5.9 (3-difluoromethyl-1-methyl-1H-pyrazole-4-carboxylic acid (9-isopropyp-1,2,3,4-tetrahydro-1,4-methano-naphthalen-5-yl)-amide) is described in WO 04/35589 and in WO 06/37632; compound B-5.10 (1,3-dimethyl-5-fluoro-1H-pyrazole-4-carboxylic acid [2-(1,3-dimethylbutyl)phenyl]-amide) is described in WO 03/10149; compound B-5.11 (3-difluoromethyl-1-methyl-1H-pyrazole-4-carboxylic acid (3',4'-dichloro-5-fluoro-1,1'-biphenyl-2-yl)-amide; "bixafen") is registered under CAS-Reg. No.: 581809-46-3 and described in WO 03/70705; compound B-5.12 (N-{2-[3-Chloro-5-(trifluoromethyl)pyridin-2-yl]ethyl}-2-(trifluoromethyl)benzamid; "fluopyram") is registered under CAS-Reg. No: 658066-35-4 and described in WO 04/16088; compounds B-5.13, B-5.14 and B-5.15 are described in WO 2007/17450; compounds B-5.16, B-5.17 and B-5.18 are described in WO 2006/120219. The compound of formula B-7.1 is described in WO 03/015519, the compound of formula B-7.2 is described in WO 2004/067528, the compound of formula B-7.3 is described in WO 2007/115644. The compound of formula V is described in WO 2001/094339. Isopyrazam (3-(difluoromethyl)-1-methyl-*N*-[1,2,3,4-tetrahydro-9-(1-methylethyl)-1,4-methanonaphthalen-5-yl]-1*H*-pyrazole-4-carboxamide) is described in WO 2004/035589, in WO 2006/037632 and in EP 1556385 and is registered under the CAS-Reg. 881685-58-1. Sedaxane (*N*-[2-[1,1'-bicyclopropyl]-2-ylphenyl]-3-(difluoromethyl)-1-methyl-1*H*-pyrazole-4-carboxamide)is described in WO 2003/074491 and is registered under the CAS-Reg. 874967-67-6; The compound of formula (VI) is described in WO 2008/014870; and the compounds of formula (VII) is described in WO 2007/048556. Fomesafen is registered under the CAS-Reg. No. 72178-02-0. 1-[4-[4-[(5S)5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone and 1-[4-[4-[5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone [1003318-67-9] are both disclosed in WO 2010/123791, WO 2008/013925, WO 2008/013622 and WO 2011/051243 page 20), 3-difluoromethyl-1-methyl-1H-pyrazole-4-carboxylic acid (3',4',5'-trifluoro-biphenyl-2-yl)-amide is dislosed in WO 2006/087343.

Throughout this document the expression "composition" stands for the various mixtures or combinations of components (A) and (B), for example in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the components (A) and (B) is not essential for working the present invention.

The compositions disclosed herein may also comprise more than one of the active components (B), if, for example, a broadening of the spectrum of disease control is desired. For instance, it may be advantageous in the agricultural practice to combine two or three components (B) with component (A). An example is a composition comprising the compound of formula (I), azoxystrobin and cyproconazole.

The following mixtures of component (A) with components (B) are preferred (the abbreviation "TX" means: "one compound selected from the group consisting of the compounds specifically described in Tables 1 and 2 of the present invention"):
(B1) a strobilurin fungicide + TX,
(B2) an azole fungicide + TX,
(B3) a morpholine fungicide + TX,
(B4) an anilinopyrimidine fungicide + TX,
(B5) a fungicide selected from the group consisting of
   Anilazine + TX, arsenates + TX, benalaxyl + TX, benalaxyl-M + TX, benodanil + TX, benomyl + TX, benthiavalicarb + TX, benthiavalicarb-isopropyl + TX, biphenyl + TX, bitertanol + TX, blasticidin-S + TX, bordeaux mixture + TX, boscalid + TX, bupirimate + TX, cadmium chloride + TX, captafol + TX, captan + TX, carbendazim + TX, carbon disulfide + TX, carboxin + TX, carpropamid + TX, cedar leaf oil + TX, chinomethionat + TX, chlorine + TX, chloroneb + TX, chlorothalonil + TX, chlozolinate + TX, cinnamaldehyde + TX, copper + TX, copper ammoniumcarbonate + TX, copper hydroxide + TX, copper octanoate + TX, copper oleate + TX, copper sulphate + TX, cyazofamid + TX, cycloheximide + TX, cymoxanil + TX, dichlofluanid + TX, dichlone + TX, dichloropropene + TX, diclocymet + TX, diclomezine + TX, dicloran + TX, diethofencarb + TX, diflumetorim + TX, dimethirimol + TX, dimethomorph + TX, dinocap + TX, dithianon + TX, dodine + TX, edifenphos + TX, ethaboxam + TX, ethirimol + TX, etridiazole + TX, famoxadone + TX, fenamidone + TX, fenaminosulf + TX, fenamiphos + TX, fenarimol + TX, fenfuram + TX, fenhexamid + TX, fenoxanil + TX, fenpiclonil + TX, fentin acetate + TX, fentin chloride + TX, fentin hydroxide + TX, ferbam + TX, ferimzone + TX, fluazinam + TX, fludioxonil + TX, flusulfamide + TX, flusulfamide + TX, flutolanil + TX, folpet + TX, formaldehyde + TX, fosetyl-aluminium + TX, fthalide + TX, fuberidazole + TX, furalaxyl + TX, furametpyr + TX, flyodin + TX, fuazatine + TX, hexachlorobenzene + TX, hymexazole + TX, iminoctadine + TX, iodocarb + TX, iprobenfos + TX, iprodione + TX, iprovalicarb + TX, isoprothiolane + TX, kasugamycin + TX, mancozeb + TX, maneb + TX, manganous dimethyldithiocarbamate + TX, mefenoxam + TX, mepronil + TX, mercuric chloride + TX, mercury + TX, metalaxyl + TX, methasulfocarb + TX, metiram + TX, metrafenone + TX, nabam + TX, neem oil (hydrophobic extract) + TX, nuarimol + TX, octhilinone + TX, ofurace + TX, oxadixyl + TX, oxine copper + TX, oxolinic acid + TX, oxycarboxin + TX, oxytetracycline + TX, paclobutrazole + TX, paraffin oil + TX, paraformaldehyde + TX, pencycuron + TX, pentachloronitrobenzene + TX, pentachlorophenol + TX, penthiopyrad + TX, perfurazoate + TX, phosphoric acid + TX, polyoxin + TX, polyoxin D zinc salt + TX, potassium bicarbonate + TX, probenazole + TX, procymidone + TX, propamocarb + TX, propineb + TX, proquinazid + TX, prothiocarb + TX, pyrazophos + TX, pyrifenox + TX, pyroquilon + TX, quinoxyfen + TX, quintozene + TX, silthiofam + TX, sodium bicarbonate + TX, sodium diacetate + TX, sodium propionate + TX, streptomycin + TX, sulphur + TX, TCMTB + TX, tecloftalam + TX, tecnazene + TX, thiabendazole + TX, thifluzamide + TX, thiophanate + TX, thiophanate-methyl + TX, thiram + TX, tolclofos-methyl + TX, tolyfluanid + TX, triazoxide + TX, trichoderma harzianum + TX, tricyclazole + TX, triforine + TX, triphenyltin hydroxide + TX, validamycin + TX, vinclozolin + TX, zineb + TX, ziram + TX, zoxamide + TX, 1 + TX,1-bis(4-chlorophenyl)-2-ethoxyethanol + TX, 2 + TX,4-dichlorophenyl benzenesulfonate + TX, 2-fluoro-*N*-methyl-*N*-1-naphthylacetamide + TX, 4-chlorophenyl phenyl sulfone + TX,
   a compound of formula B-5.1 + TX 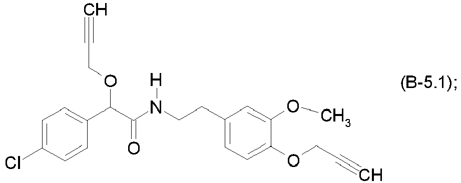
   a compound of formula B-5.2 + TX 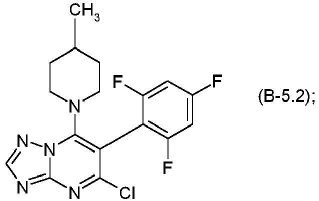
   a compound of formula B-5.3 + TX 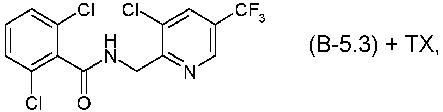
   a compound of formula B-5.4 + TX 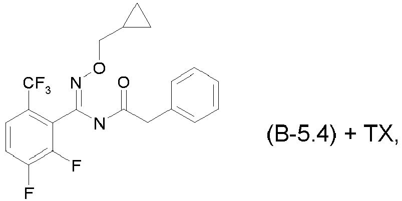
   a compound of formula B-5.5 + TX 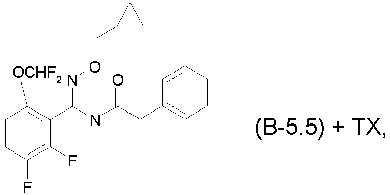
   a compound of formula B-5.6 + TX 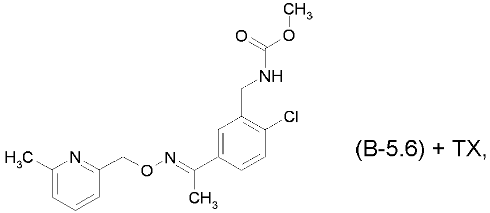
   a compound of formula B-5.7 + TX 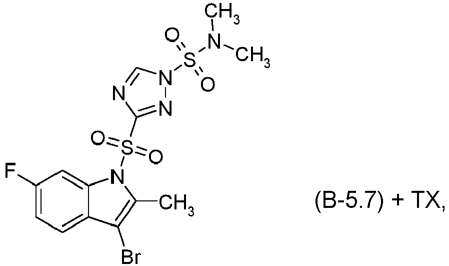
   3-difluoromethyl-1-methyl-1H-pyrazole-4-carboxylic acid (2-bicyclopropyl-2-yl-phenyl)-amide (compound B-5.8) + TX, 3-difluoromethyl-1-methyl-1H-pyrazole-4-carboxylic acid (9-isopropyp-1,2,3,4-tetrahydro-1,4-methano-naphthalen-5-yl)-amide (compound B-5.9) + TX, 1,3-dimethyl-5-fluoro-1H-pyrazole-4-carboxylic acid [2-(1,3-dimethylbutyl)phenyl]-amide (compound B-5.10) + TX, 3-difluoromethyl-1-methyl-1H-pyrazole-4-carboxylic acid (3',4'-dichloro-5-fluoro-1,1'-biphenyl-2-yl)-amide (compound B-5.11) + TX, N-{2-[3-chloro-5-(trifluoromethyl)pyridin-2-yl]ethyl}-2-(trifluoromethyl)benzamid (compound B-5.12) + TX, 3-difluoromethyl-1-methyl-1H-pyrazole-4-carboxylic acid N-[2-(1,1,2,2-tetrafluoroethoxy)phenyl]-amide (compound B-5.13) + TX, 3-difluoromethyl-1-methyl-1H-pyrazole-4-carboxylic acid N-[2-(1,1,2,3,3,3-hexafluoropropoxy)phenyl]-amide (compound B-5.14), 3-difluoromethyl-1-methyl-1H-pyrazole-4-carboxylic acid N-[2-(2-chloro-1 + TX,1,2-trifluoroethoxy)phenyl]-amide (compound B-5.15) + TX, 3-difluoromethyl-1-methyl-1H-pyrazole-4-carboxylic acid N-(4'-trifluoromethyl-biphen-2-yl)-amide (compound B-5.16) + TX, 3-difluoromethyl-1-methyl-1H-pyrazole-4-carboxylic acid N-(2'-trifluoromethyl-biphen-2-yl)-amide (compound B-5.17) and 3-difluoromethyl-1-methyl-1H-pyrazole-4-carboxylic acid N-(2'-trifluoromethyl-biphen-2-yl)-amide (compound B-5.18) + TX; (B6) a plant-bioregulator selected from the group consisting of
   acibenzolar-S-methyl + TX, chlormequat chloride + TX, ethephon + TX, mepiquat chloride and trinexapac-ethyl;
(B7) an insecticide selected from the group consisting of
   abamectin + TX, clothianidin + TX, emamectin benzoate + TX, imidacloprid + TX, tefluthrin + TX, thiamethoxam + TX,
   a compound of formula B-7.1 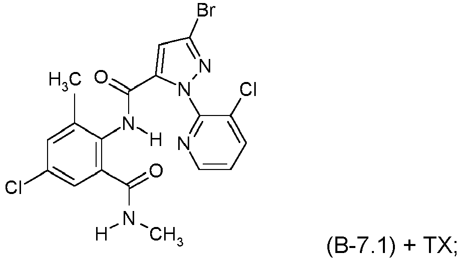
   a compound of formula B-7.2; 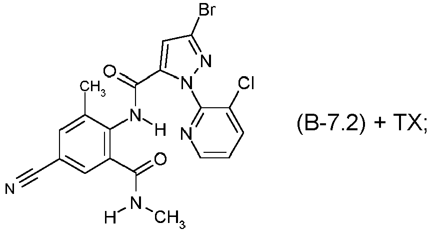 and
   a compound of formula B-7.3 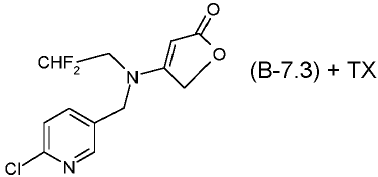 and
(B8) glyphosate + TX, a compound of formula V + TX 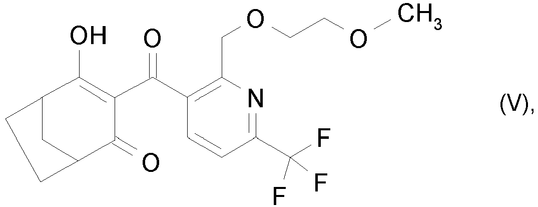
   fomesafen + TX, and (B9) isopyrazam + TX, sedaxane + TX,
   a compound of formula (VI) + TX 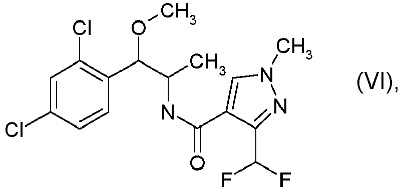
   a compound of formula (VII) + TX 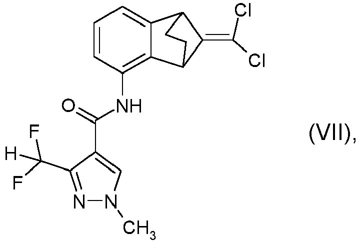
   1-[4-[4-[(5S)5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone + TX, 1-[4-[4-[5-(2,6-difluorophenyl)-4,5-dihydro-1,2-oxazol-3-yl]-1,3-thiazol-2-yl]piperidin-1-yl]-2-[5-methyl-3-(trifluoromethyl)-1H-pyrazol-1-yl]ethanone +TX, and 3-difluoromethyl-1-methyl-1H-pyrazole-4-carboxylic acid (3',4',5'-trifluoro-biphenyl-2-yl)-amide + TX.

The following mixtures of component (A) with components (B) are especially preferred:
(B1) a strobilurin fungicide + TX,
(B2) an azole fungicide + TX,
(B3) a morpholine fungicide + TX,
(B4) an anilinopyrimidine fungicide + TX,
(B5) a fungicide selected from the group consisting of
   anilazine (878) + TX, arsenates + TX, benalaxyl (56) + TX, benalaxyl-M + TX, benodanil (896) + TX, benomyl (62) + TX, benthiavalicarb + TX, benthiavalicarb-isopropyl (68) + TX, biphenyl (81) + TX, bitertanol (84) + TX, blasticidin-S (85) + TX, bordeaux mixture (87) + TX, boscalid (88) + TX, bupirimate (98) + TX, cadmium chloride + TX, captafol (113) + TX, captan (114) + TX, carbendazim (116) + TX, carbon disulfide (945) + TX, carboxin (120) + TX, carpropamid (122) + TX, cedar leaf oil + TX, chinomethionat (126) + TX, chlorine + TX, chloroneb (139) + TX, chlorothalonil (142) + TX, chlozolinate (149) + TX, cinnamaldehyde + TX, copper + TX, copper ammoniumcarbonate + TX, copper hydroxide (169) + TX, copper octanoate (170) + TX, copper oleate + TX, copper sulphate (87) + TX, cyazofamid (185) + TX, cycloheximide (1022) + TX, cymoxanil (200) + TX, dichlofluanid (230) + TX, dichlone (1052) + TX, dichloropropene (233) + TX, diclocymet (237) + TX, diclomezine (239) + TX, dicloran (240) + TX, diethofencarb (245) + TX, diflumetorim (253) + TX, dimethirimol (1082) + TX, dimethomorph (263) + TX, dinocap (270) + TX, dithianon (279) + TX, dodine (289) + TX, edifenphos (290) + TX, ethaboxam (304) + TX, ethirimol (1133) + TX, etridiazole (321) + TX, famoxadone (322) + TX, fenamidone (325) + TX, fenaminosulf (1144) + TX, fenamiphos (326) + TX, fenarimol (327) + TX, fenfuram (333) + TX, fenhexamid (334) + TX, fenoxanil (338) + TX, fenpiclonil (341) + TX, fentin acetate (347) + TX, fentin chloride + TX, fentin hydroxide (347) + TX, ferbam (350) + TX, ferimzone (351) + TX, fluazinam (363) + TX, fludioxonil (368) + TX, flusulfamide (394) + TX, flutolanil (396) + TX, folpet (400) + TX, formaldehyde (404) + TX, fosetyl-aluminium (407) + TX, fthalide (643) + TX, fuberidazole (419) + TX, furalaxyl (410) + TX, furametpyr (411) + TX, flyodin (1205) + TX, fuazatine (422) + TX, hexachlorobenzene (434) + TX, hymexazole + TX, iminoctadine (459) + TX, iodocarb (3-lodo-2-propynyl butyl carbamate) + TX, iprobenfos (IBP) (469) + TX, iprodione (470) + TX, iprovalicarb (471) + TX, isoprothiolane (474) + TX, kasugamycin (483) + TX, mancozeb (496) + TX, maneb (497) + TX, manganous dimethyldithiocarbamate + TX, mefenoxam (Metalaxyl-M) (517) + TX, mepronil (510) + TX, mercuric chloride (511) + TX, mercury + TX, metalaxyl (516) + TX, methasulfocarb (528) + TX, metiram (546) + TX, metrafenone + TX, nabam (566) + TX, neem oil (hydrophobic extract) + TX, nuarimol (587) + TX, octhilinone (590) + TX, ofurace (592) + TX, oxadixyl (601) + TX, oxine copper (605) + TX, oxolinic acid (606) + TX, oxycarboxin (608) + TX, oxytetracycline (611) + TX, paclobutrazole (612) + TX, paraffin oil (628) + TX, paraformaldehyde + TX, pencycuron (620) + TX, pentachloronitrobenzene (716) + TX, pentachlorophenol (623) + TX, penthiopyrad + TX, perfurazoate + TX, phosphoric acid + TX, polyoxin (654) + TX, polyoxin D zinc salt (654) + TX, potassium bicarbonate + TX, probenazole (658) + TX, procymidone (660) + TX, propamocarb (668) + TX, propineb (676) + TX, proquinazid (682) + TX, prothiocarb (1361) + TX, pyrazophos (693) + TX, pyrifenox (703) + TX, pyroquilon (710) + TX, quinoxyfen (715) + TX, quintozene (PCN(B) (716) + TX, silthiofam (729) + TX, sodium bicarbonate + TX, sodium diacetate + TX, sodium propionate + TX, streptomycin (744) + TX, sulphur (754) + TX, TCMTB + TX, tecloftalam + TX, tecnazene (TCN(B) (767) + TX, thiabendazole (790) + TX, thifluzamide (796) + TX, thiophanate (1435) + TX, thiophanate-methyl (802) + TX, thiram (804) + TX, tolclofos-methyl (808) + TX, tolylfluanid (810) + TX, triazoxide (821) + TX, trichoderma harzianum (825) + TX, tricyclazole (828) + TX, triforine (838) + TX, triphenyltin hydroxide (347) + TX, validamycin (846) + TX, vinclozolin (849) + TX, zineb (855) + TX, ziram (856) + TX, zoxamide (857) + TX, 1,1-bis(4-chlorophenyl)-2-ethoxyethanol (IUPAC-Name) (910) + TX, 2 + TX,4-dichlorophenyl benzenesulfonate (IUPAC- / Chemical Abstracts-Name) (1059) + TX, 2-fluoro-*N*-methyl-*N*-1-naphthylacetamide (IUPAC-Name) (1295) + TX, 4-chlorophenyl phenyl sulfone (IUPAC-Name) (981) + TX,
   a compound of formula B-5.1 +TX 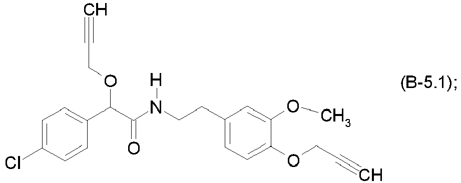
   a compound of formula B-5.2 +TX 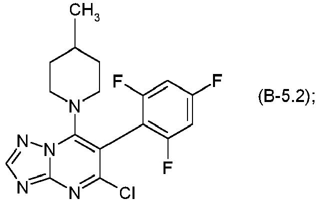
   a compound of formula B-5.3 +TX 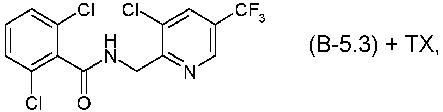
   a compound of formula B-5.4 +TX 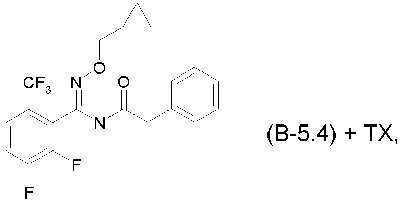
   a compound of formula B-5.5 +TX 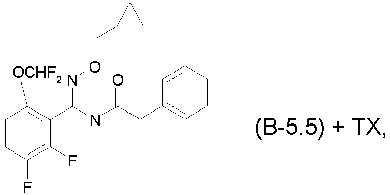
   a compound of formula B-5.6 +TX 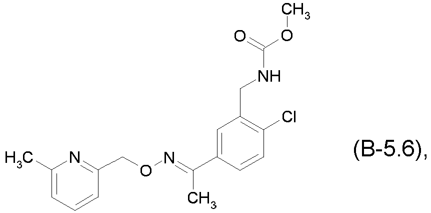
   a compound of formula B-5.7 +TX 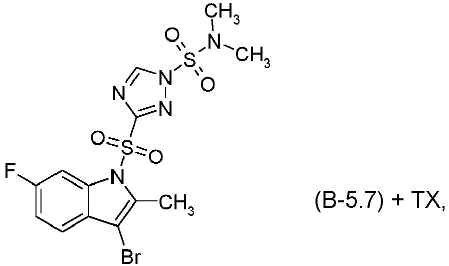
   3-difluoromethyl-1-methyl-1H-pyrazole-4-carboxylic acid (2-bicyclopropyl-2-yl-phenyl)-amide (compound B-5.8) + TX, 3-difluoromethyl-1-methyl-1H-pyrazole-4-carboxylic acid (9-isopropyp-1,2,3,4-tetrahydro-1,4-methano-naphthalen-5-yl)-amide (compound B-5.9) + TX, 1,3-dimethyl-5-fluoro-1H-pyrazole-4-carboxylic acid [2-(1,3-dimethylbutyl)phenyl]-amide (compound B-5.10) + TX, 3-difluoromethyl-1-methyl-1H-pyrazole-4-carboxylic acid (3',4'-dichloro-5-fluoro-1,1'-biphenyl-2-yl)-amide (compound B-5.11) + TX, N-{2-[3-chloro-5-(trifluoromethyl)pyridin-2-yl]ethyl}-2-(trifluoromethyl)benzamid (compound B-5.12) + TX, 3-difluoromethyl-1-methyl-1H-pyrazole-4-carboxylic acid N-[2-(1,1,2,2-tetrafluoroethoxy)phenyl]-amide (compound B-5.13) + TX, 3-difluoromethyl-1-methyl-1H-pyrazole-4-carboxylic acid N-[2-(1,1,2,3,3,3-hexafluoropropoxy)phenyl]-amide (compound B-5.14) + TX, 3-difluoromethyl-1-methyl-1H-pyrazole-4-carboxylic acid N-[2-(2-chloro-1,1,2-trifluoroethoxy)phenyl]-amide (compound B-5.15) + TX, 3-difluoromethyl-1-methyl-1H-pyrazole-4-carboxylic acid N-(4'-trifluoromethyl-biphen-2-yl)-amide (compound B-5.16) + TX, 3-difluoromethyl-1-methyl-1H-pyrazole-4-carboxylic acid N-(2'-trifluoromethyl-biphen-2-yl)-amide (compound B-5.17) and 3-difluoromethyl-1-methyl-1H-pyrazole-4-carboxylic acid N-(2'-trifluoromethyl-biphen-2-yl)-amide (compound B-5.18);
(B6) a plant-bioregulator selected from the group consisting of
   acibenzolar-S-methyl (6) + TX, chlormequat chloride (137) + TX, ethephon (307) + TX, mepiquat chloride (509) and trinexapac-ethyl (841);
(B7) an insecticide selected from the group consisting of
   abamectin (1) + TX, clothianidin (165) + TX, emamectin benzoate (291) + TX, imidacloprid (458) + TX, tefluthrin (769) + TX, thiamethoxam (792) + TX, a compound of formula B-7.1 +TX 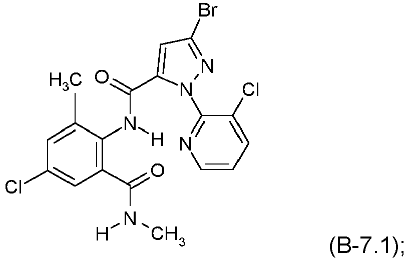
   a compound of formula B-7.2 +TX; 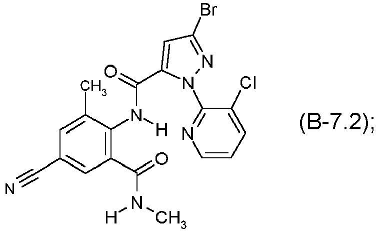
   a compound of formula B-7.3 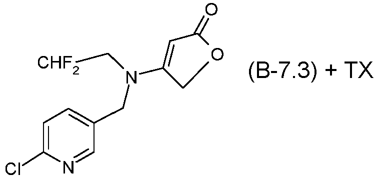 and
(B8) glyphosate (419) +TX.

Examples of especially suitable mixtures selected from the following group P:
Group P: especially suitable mixtures:
   a strobilurin fungicide selected from azoxystrobin (47) + TX, dimoxystrobin (226) + TX, fluoxastrobin (382) + TX, kresoxim-methyl (485) + TX, metominostrobin (551) + TX, orysastrobin + TX, picoxystrobin (647) + TX, pyraclostrobin (690); trifloxystrobin (832) + TX, a compound of formula B-1.1 +TX 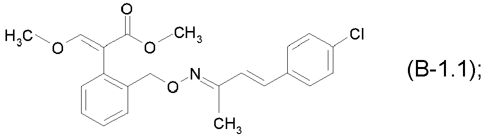
   an azole fungicide selected from azaconazole (40) + TX, bromuconazole (96) + TX, cyproconazole (207) + TX, difenoconazole (247) + TX, diniconazole (267) + TX, diniconazole-M (267) + TX, epoxiconazole (298) + TX, fenbuconazole (329) + TX, fluquinconazole (385) + TX, flusilazole (393) + TX, flutriafol (397) + TX, hexaconazole (435) + TX, imazalil (449) + TX, imibenconazole (457) + TX, ipconazole (468) + TX, metconazole (525) + TX, myclobutanil (564) + TX, oxpoconazole (607) + TX, pefurazoate (618) + TX, penconazole (619) + TX, prochloraz (659) + TX, propiconazole (675) + TX, prothioconazole (685) + TX, simeconazole (731) + TX, tebuconazole (761) + TX, tetraconazole (778) + TX, triadimefon (814) + TX, triadimenol (815) + TX, triflumizole (834) + TX, triticonazole (842) + TX, diclobutrazol (1068) + TX, etaconazole (1129) + TX, furconazole (1198) + TX, furconazole-cis (1199) and quinconazole (1378);
   a morpholine fungicide mixture selected from aldimorph + TX, dodemorph (288) + TX, fenpropimorph (344) + TX, tridemorph (830) + TX, fenpropidin (343) + TX, spiroxamine (740) + TX, piperalin (648) and a compound of formula B-3.1 +TX 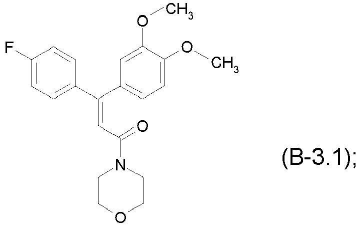
   an anilino-pyrimidine fungicide selected from cyprodinil (208) + TX, mepanipyrim (508) and pyrimethanil (705);
   a fungicide mixture selected from the group consisting of
   anilazine (878) + TX, arsenates + TX, benalaxyl (56) + TX, benalaxyl-M + TX, benodanil (896) + TX, benomyl (62) + TX, benthiavalicarb + TX, benthiavalicarb-isopropyl (68) + TX, biphenyl (81) + TX, bitertanol (84) + TX, blasticidin-S (85) + TX, bordeaux mixture (87) + TX, boscalid (88) + TX, bupirimate (98) + TX, cadmium chloride + TX, captafol (113) + TX, captan (114) + TX, carbendazim (116) + TX, carbon disulfide (945) + TX, carboxin (120) + TX, carpropamid (122) + TX, cedar leaf oil + TX, chinomethionat (126) + TX, chlorine + TX, chloroneb (139) + TX, chlorothalonil (142) + TX, chlozolinate (149) + TX, cinnamaldehyde + TX, copper + TX, copper ammoniumcarbonate + TX, copper hydroxide (169) + TX, copper octanoate (170) + TX, copper oleate + TX, copper sulphate (87) + TX, cyazofamid (185) + TX, cycloheximide (1022) + TX, cymoxanil (200) + TX, dichlofluanid (230) + TX, dichlone (1052) + TX, dichloropropene (233) + TX, diclocymet (237) + TX, diclomezine (239) + TX, dicloran (240) + TX, diethofencarb (245) + TX, diflumetorim (253) + TX, dimethirimol (1082) + TX, dimethomorph (263) + TX, dinocap (270) + TX, dithianon (279) + TX, dodine (289) + TX, edifenphos (290) + TX, ethaboxam (304) + TX, ethirimol (1133) + TX, etridiazole (321) + TX, famoxadone (322) + TX, fenamidone (325) + TX, fenaminosulf (1144) + TX, fenamiphos (326) + TX, fenarimol (327) + TX, fenfuram (333) + TX, fenhexamid (334) + TX, fenoxanil (338) + TX, fenpiclonil (341) + TX, fentin acetate (347) + TX, fentin chloride + TX, fentin hydroxide (347) + TX, ferbam (350) + TX, ferimzone (351) + TX, fluazinam (363) + TX, fludioxonil (368) + TX, flusulfamide (394) + TX, flutolanil (396) + TX, folpet (400) + TX, formaldehyde (404) + TX, fosetyl-aluminium (407) + TX, fthalide (643) + TX, fuberidazole (419) + TX, furalaxyl (410) + TX, furametpyr (411) + TX, flyodin (1205) + TX, fuazatine (422) + TX, hexachlorobenzene (434) + TX, hymexazole + TX, iminoctadine (459) + TX, iodocarb (3-lodo-2-propynyl butyl carbamate) + TX, iprobenfos (IBP) (469) + TX, iprodione (470) + TX, iprovalicarb (471) + TX, isoprothiolane (474) + TX, kasugamycin (483) + TX, mancozeb (496) + TX, maneb (497) + TX, manganous dimethyldithiocarbamate + TX, mefenoxam (Metalaxyl-M) (517) + TX, mepronil (510) + TX, mercuric chloride (511) + TX, mercury + TX, metalaxyl (516) + TX, methasulfocarb (528) + TX, metiram (546) + TX, metrafenone + TX, nabam (566) + TX, neem oil (hydrophobic extract) + TX, nuarimol (587) + TX, octhilinone (590) + TX, ofurace (592) + TX, oxadixyl (601) + TX, oxine copper (605) + TX, oxolinic acid (606) + TX, oxycarboxin (608) + TX, oxytetracycline (611) + TX, paclobutrazole (612) + TX, paraffin oil (628) + TX, paraformaldehyde + TX, pencycuron (620) + TX, pentachloronitrobenzene (716) + TX, pentachlorophenol (623) + TX, penthiopyrad + TX, perfurazoate + TX, phosphoric acid + TX, polyoxin (654) + TX, polyoxin D zinc salt (654) + TX, potassium bicarbonate + TX, probenazole (658) + TX, procymidone (660) + TX, propamocarb (668) + TX, propineb (676) + TX, proquinazid (682) + TX, prothiocarb (1361) + TX, pyrazophos (693) + TX, pyrifenox (703) + TX, pyroquilon (710) + TX, quinoxyfen (715) + TX, quintozene (PCN(B) (716) + TX, silthiofam (729) + TX, sodium bicarbonate + TX, sodium diacetate + TX, sodium propionate + TX, streptomycin (744) + TX, sulphur (754) + TX, TCMTB + TX, tecloftalam + TX, tecnazene (TCN(B) (767) + TX, thiabendazole (790) + TX, thifluzamide (796) + TX, thiophanate (1435) + TX, thiophanate-methyl (802) + TX, thiram (804) + TX, tolclofos-methyl (808) + TX, tolylfluanid (810) + TX, triazoxide (821) + TX, trichoderma harzianum (825) + TX, tricyclazole (828) + TX, triforine (838) + TX, triphenyltin hydroxide (347) + TX, validamycin (846) + TX, vinclozolin (849) + TX, zineb (855) + TX, ziram (856) + TX, zoxamide (857) + TX, 1 + TX,1-bis(4-chlorophenyl)-2-ethoxyethanol (IUPAC-Name) (910) + TX, 2 + TX,4-dichlorophenyl benzenesulfonate (IUPAC- / Chemical Abstracts-Name) (1059) + TX, 2-fluoro-*N*-methyl-*N*-1-naphthylacetamide (IUPAC-Name) (1295) + TX, 4-chlorophenyl phenyl sulfone (IUPAC-Name) (981) + TX,
   a compound of formula B-5.1 + TX, a compound of formula B-5.2 + TX, a compound of formula B-5.3 + TX, a compound of formula B-5.4 + TX, a compound of formula B-5.5 + TX, a compound of formula B-5.6 + TX, a compound of formula B-5.7 + TX, compound B-5.8 + TX, compound B-5.9 + TX, compound B-5.10 + TX, compound B-5.11 + TX, compound B-5.12 + TX, compound B-5.13 + TX, compound B-5.14 + TX, compound B-5.15 + TX, compound B-5.16 + TX, compound B-5.17 and compound B-5.18;
   a plant-bioregulator selected from the group consisting of
   acibenzolar-S-methyl (6) + TX, chlormequat chloride (137) + TX, ethephon (307) + TX, mepiquat chloride (509) and trinexapac-ethyl (841);
   an insecticide selected from the group consisting of
   abamectin (1) + TX, clothianidin (165) + TX, emamectin benzoate (291) + TX, imidacloprid (458) + TX, tefluthrin (769) + TX, thiamethoxam (792) + TX, and glyphosate (419) + TX, a compound of formula V 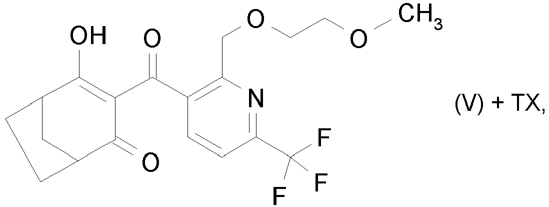
   fomesafen + TX, and (B9) isopyrazam + TX, sedaxane + TX,
   a compound of formula (VI) + TX 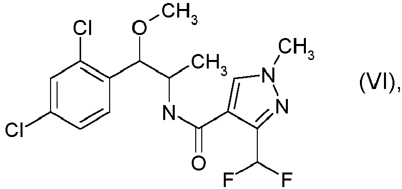 and
   a compound of formula (VII) + TX 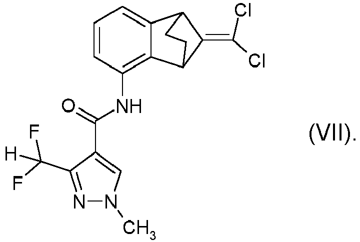 Further examples of especially suitable mixtures selected from the following group Q:
Group Q: especially suitable compositions:
   a strobilurin fungicide selected from the group consisting of azoxystrobin + TX, dimoxystrobin + TX, fluoxastrobin + TX, kresoxim-methyl + TX, metominostrobin + TX, orysastrobin + TX, picoxystrobin + TX, pyraclostrobin; trifloxystrobin and a compound of formula B-1.1;
   an azole fungicide selected from the group consisting of azaconazole + TX, bromuconazole + TX, cyproconazole + TX, difenoconazole + TX, diniconazole + TX, diniconazole-M + TX, epoxiconazole + TX, fenbuconazole + TX, fluquinconazole + TX, flusilazole + TX, flutriafol + TX, hexaconazole + TX, imazalil + TX, imibenconazole + TX, ipconazole + TX, metconazole + TX, myclobutanil + TX, oxpoconazole + TX, pefurazoate + TX, penconazole + TX, prochloraz + TX, propiconazole + TX, prothioconazole + TX, simeconazole + TX, tebuconazole + TX, tetraconazole + TX, triadimefon + TX, triadimenol + TX, triflumizole + TX, triticonazole + TX, diclobutrazol + TX, etaconazole + TX, furconazole + TX, furconazole-cis and quinconazole;
   a morpholine fungicide selected from the group consisting of aldimorph + TX, dodemorph + TX, fenpropimorph + TX, tridemorph + TX, fenpropidin + TX, spiroxamine + TX, piperalin and a compound of formula B-3.1;
   an anilino-pyrimidine fungicide selected from the group consisting of cyprodinil + TX, mepanipyrim and pyrimethanil;
   a fungicide selected from the group consisting of benalaxyl + TX, benalaxyl-M + TX, benomyl + TX, bitertanol + TX, boscalid + TX, captan + TX, carboxin + TX, carpropamid + TX, chlorothalonil + TX, copper + TX, cyazofamid + TX, cymoxanil + TX, diethofencarb + TX, dithianon + TX, famoxadone + TX, fenamidone + TX, fenhexamide + TX, fenoxycarb + TX, fenpiclonil + TX, fluazinam + TX, fludioxonil + TX, flutolanil + TX, folpet + TX, guazatine + TX, hymexazole + TX, iprodione + TX, lufenuron + TX, mancozeb + TX, metalaxyl + TX, mefenoxam + TX, metrafenone + TX, nuarimol + TX, paclobutrazol + TX, pencycuron + TX, penthiopyrad + TX, procymidone + TX, proquinazid + TX, pyroquilon + TX, quinoxyfen + TX, silthiofam + TX, sulfur + TX, thiabendazole + TX, thiram + TX, triazoxide + TX, tricyclazole + TX, a compound of formula B-5.1 + TX, a compound of formula B-5.2 + TX, a compound of formula B-5.3 + TX, a compound of formula B-5.4 + TX, a compound of formula B-5.5 + TX, a compound of formula B-5.6 + TX, a compound of formula B-5.7 + TX, a compound of formula B-5.8 + TX, a compound of formula B-5.9 + TX, a compound of formula B-5.10 and a compound of formula B-5.12;
   a plant-bioregulator selected from acibenzolar-S-methyl + TX, chlormequat chloride + TX, ethephon + TX, mepiquat chloride and trinexapac-ethyl;
   an insecticide selected from abamectin + TX, emamectin benzoate + TX, tefluthrin + TX, thiamethoxam + TX, and glyphosate + TX, a compound of formula V 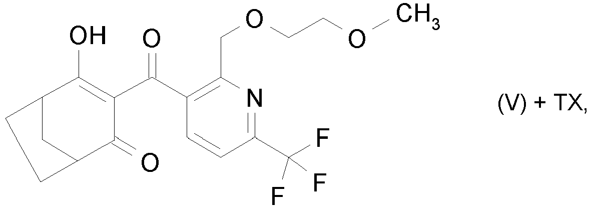
   fomesafen + TX, and (B9) isopyrazam + TX, sedaxane + TX,
   a compound of formula (VI) + TX 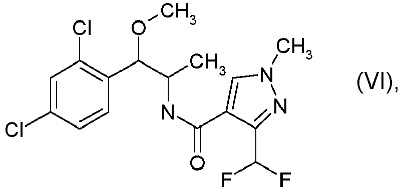 and
   a compound of formula (VII) + TX

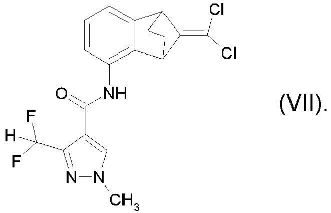

The active ingredient mixture of the compound of formula I with active ingredients (B) described above comprises the compound of formula I and an active ingredient as described above preferably in a mixing ratio of from 1000:1 to 1:1000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are understood to include, on the one hand, ratios by weight and also, on other hand, molar ratios.

The mixtures comprising a compound of formula I and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compound of formula I and the active ingredients as described above is not essential.

When applied to the useful plants the compound of formula I is applied at a rate of 5 to 2000 g a.i./ha, particularly 10 to 1000 g a.i./ha, e.g. 50, 75, 100 or 200 g a.i./ha, in association with 1 to 5000 g a.i./ha, particularly 2 to 2000 g a.i./ha, e.g. 100, 250, 500, 800, 1000, 1500 g a.i./ha of a compound of component (B), depending on the class of chemical employed as component (B).

In agricultural practice the application rates of the combinations disclosed herein depend on the type of effect desired, and typically range from 20 to 4000 g of total combination per hectare.

When the combinations disclosed herein are used for treating seed, rates of 0.001 to 50 g of the compound of formula I per kg of seed, preferably from 0.01 to 10g per kg of seed, and 0.001 to 50 g of a compound of component (B), per kg of seed, preferably from 0.01 to 10g per kg of seed, are generally sufficient.

The compositions disclosed herein may be employed in any conventional form, for example in the form of a twin pack, a powder for dry seed treatment (DS), an emulsion for seed treatment (ES), a flowable concentrate for seed treatment (FS), a solution for seed treatment (LS), a water dispersible powder for seed treatment (WS), a capsule suspension for seed treatment (CF), a gel for seed treatment (GF), an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP) or any technically feasible formulation in combination with agriculturally acceptable adjuvants.

Such compositions may be produced in conventional manner, e.g. by mixing the active ingredients with appropriate formulation inerts (diluents, solvents, fillers and optionally other formulating ingredients such as surfactants, biocides, anti-freeze, stickers, thickeners and compounds that provide adjuvancy effects). Also conventional slow release formulations may be employed where long lasting efficacy is intended. Particularly formulations to be applied in spraying forms, such as water dispersible concentrates (e.g. EC, SC, DC, OD, SE, EW, EO and the like), wettable powders and granules, may contain surfactants such as wetting and dispersing agents and other compounds that provide adjuvancy effects, e.g. the condensation product of formaldehyde with naphthalene sulphonate, an alkylarylsulphonate, a lignin sulphonate, a fatty alkyl sulphate, and ethoxylated alkylphenol and an ethoxylated fatty alcohol.

A seed dressing formulation is applied in a manner known per se to the seeds employing the combination of the invention and a diluent in suitable seed dressing formulation form, e.g. as an aqueous suspension or in a dry powder form having good adherence to the seeds. Such seed dressing formulations are known in the art. Seed dressing formulations may contain the single active ingredients or the combination of active ingredients in encapsulated form, e.g. as slow release capsules or microcapsules.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula I together with a compound of component (B), and optionally other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

The Examples which follow serve to illustrate the invention, "active ingredient" denoting a mixture of compound I and a compound of component (B) in a specific mixing ratio.

### Formulation Examples

| Wettable powders | a) | b) | c) |
|---|---|---|---|
| active ingredient [I : comp (B) = 1:3(a), 1:2(b), 1:1(c)] | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether (7-8 mol of ethylene oxide) | - | 2 % | - |
| highly dispersed silicic acid | 5 % | 10 % | 10 % |
| Kaolin | 62 % | 27 % | - |

The active ingredient is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredient [I : comp (B) = 1:3(a), 1:2(b), 1:1(c)] | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 |

The active ingredient is thoroughly mixed with the adjuvants and the mixture is thoroughly ground in a suitable mill, affording powders that can be used directly for seed treatment.

| Emulsifiable concentrate | |
|---|---|
| active ingredient (I : comp (B) = 1:6) | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredient [I : comp (B) = 1:6(a), 1:2(b), 1:10(c)] | 5 % | 6 % | 4 % |
| talcum | 95 % | - | - |
| Kaolin | - | 94 % | - |
| mineral filler | - | - | 96 % |

Ready-for-use dusts are obtained by mixing the active ingredient with the carrier and grinding the mixture in a suitable mill. Such powders can also be used for dry dressings for seed.

### Extruder granules

| | |
|---|---|
| Active ingredient (I : comp (B) = 2:1) | 15 % |
| sodium lignosulfonate | 2 % |
| carboxymethylcellulose | 1 % |
| Kaolin | 82 % |

The active ingredient is mixed and ground with the adjuvants, and the mixture is moistened with water. The mixture is extruded and then dried in a stream of air.

### Coated granules

| | |
|---|---|
| Active ingredient (I :comp (B) = 1:10) | 8 % |
| polyethylene glycol (mol. wt. 200) | 3 % |
| Kaolin | 89 % |

The finely ground active ingredient is uniformly applied, in a mixer, to the kaolin moistened with polyethylene glycol. Non-dusty coated granules are obtained in this manner.

### Suspension concentrate

| | |
|---|---|
| active ingredient (I : comp (B) = 1:8) | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredient (I : comp (B) = 1:8) | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of a combination of the compound of formula I and a compound of component (B), or of each of these compounds separately, are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed.

The obtained capsule suspension is stabilized by adding 0.25 parts of a thickener and 3 parts of a dispersing agent. The capsule suspension formulation contains 28% of the active ingredients. The medium capsule diameter is 8-15 microns.

The resulting formulation is applied to seeds as an aqueous suspension in an apparatus suitable for that purpose.

### Biological Examples

### Example B1: Sensitivity of isolates of Fusarium virguliforme to the compound No. 1.001:

The sensitivity of 4 *F. virguliforme* isolates to the compound No. 1.001 was determined by comparing the mycelial growth inhibition of on potato dextrose agar plates amended or not with the compound No. 1.001. The compound No. 1.001 was tested at the following concentrations: 0, 0.001, 0.01, 0.1, 1 and 10 mg.ai./L. The fungicide fludioxonil (commercial formulation Maxim 4 FS) was included in the sensitivity study. The plates were incubated at 20 °C for 9 days. The sensitivity test was set in a completely randomized design with 2 replications for each concentration of fungicide tested. Data obtained for each isolate was transformed using an arcsine transformation. The effective dose for 50% of the fungal growth inhibition (ED₅₀ values) was calculated by regressing the transformed growth inhibition data against the log of the fungicide concentration.

The compound No. 1.001 had a very good intrinsic activity against *F. virguliforme.* One isolate was sensitive to fludioxonil. There was not a dose response of the other 3 isolates to fludioxonil.

**Table 1. Sensitivity of 4 F. virguliforme isolates to the compound No. 1.001:**

| Isolate | compound No. 1.001 ED50 (mg ai/L) | Fludioxonil ED50 (mg ai/L) |
|---|---|---|
| 10-118 | 0.163 | 0.206 |
| 10-119 | 0.137 | >10 |
| 10-120 | 0.041 | >10 |
| 10-230 | 0.117 | >10 |

### Example B2: Evaluation of the efficacy of the compound No. 1.001 against Fusarium virguliforme, the causal agent of the sudden death syndrome on soybeans:

A study was conducted to determine the efficacy of the compound No. 1.001 against *F*. *virguliforme* the causal agent of the sudden death syndrome on soybeans. Soybean seeds of the cultivar S36-B6 were treated with the compound No. 1.001 at a rate of 5 and 10 grams of active ingredient per 100 kilograms of seeds. The fungicide fludioxonil (commercial formulation Maxim 4 FS) was was also included in the test at a rate of 2.5 grams of active ingredient per 100 kilograms of seeds. Ten soybean seeds were placed on a paper towel (Scotts paper towels, 25.4 cm × 33.02 cm in size) that has been previously moistened with 40 milliliters of water. Seeds were inculated with 100 microliters of a *F. virguliforme* conidial suspension adjusted to 100,000 conidia per milliter of water. The isolate with the identification number 10-118 of *F. virguliforme* was used used in this study since it is sensitive to fludioxonil. Inoculated seeds seeds were then covered with another paper towel. Starting at one end, the paper towels with seeds were rolled loosely in a cilinder-fashion. The rolled paper towels with the inoculatede seeds were placed into a zipped plastic bag. Bags were labelled with the fungicide treatment and dated. Bags with seeds were then placed in a growth room at a temperature of 18 °C and 12 h photoperiod for 19 days. The study was conducted using a completely randomized design with 3 replications. Disease severity was conducted on the hypocotyls and roots and measured as percentage.

Disease severity in the untreated inoculated plants was 57.9% and was not detected on the untreated uninoculated plants. Disease was completed controlled with the compound No. 1.001 at the rates of 5 and 10 grams of active ingredient per 100 kilograms of seeds. Maxim 4 FS was only able to reduce the disease severity to 20.9%.

**Table 2. Control of soybean sudden death syndrome caused by Fusarium verguliforme with the compound No. 1.001:**

| Fungicide | Fungicide rate mg a.i./100 kg seed | % disease severity ab | Mean separation ^{c} |
|---|---|---|---|
| Untreated uninoculated | - | 0.0 | C |
| Untreated inoculated | - | 57.9 | A |
| Fludioxonil (Maxim 4 FS) | 2.5 | 20.9 | B |
| the compound No. 1.001 | 5 | 0.0 | C |
| the compound No. 1.001 | 10 | 0.0 | C |

| | | | |
|---|---|---|---|
| ^{a.} Mean of 3 replications ^{b.} % disease severity was conducted on the hypocotyls and roots ^{c.} % Means within a column followed by the same letter are not significant different (*p*<*0.001*) | | | |

## Claims

1. A method for reducing the occurrence of phytopathogenic fungi selected from the group consisting of: *Fusarium virguliforme, Fusarium brasiliense* sp. nov., *Fusarium cuneirostrum* sp. nov. and *Fusarium tucumaniae,* in soybean plants, said method comprising applying an effective amount of a composition comprising the compound of formula (I) 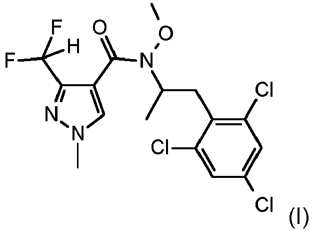
or an agrochemically acceptable salt, isomer, stereoisomer, diasterioisomer, enantiomer or tautomer thereof,
to a soybean plant seed prior to planting,
wherein said soybean plant seed is infected with a fungal strain selected from the group consisting of *Fusarium virguliforme, Fusarium brasiliense* sp. nov., *Fusarium cuneirostrum* sp. nov. and *Fusarium tucumaniae.*

2. A method for suppressing, controlling or reducing Soybean Sudden Death Syndrome in soybean plants comprising planting in soil a soybean plant seed treated with the compound of formula (I) as defined in claim 1, wherein said soybean plant seed contains a precursor to Soybean Sudden Death Syndrome, said precursor selected from the group consisting of *Fusarium brasiliense* sp. nov., *Fusarium cuneirostrum* sp. nov. and *Fusarium tucumaniae.*

3. A method for reducing damage to a soybean plant or soybean plant seed caused by Soybean Sudden Death Syndrome comprising applying to a soybean plant seed prior to planting, the compound of formula (I) as defined in claim 1, wherein said soybean plant seed contains a precursor to Sudden Death Syndrome selected from the group consisting of *Fusarium brasiliense* sp. nov., *Fusarium cuneirostrum* sp. nov. and *Fusarium tucumaniae;* and planting said soybean plant seed.

4. The method of claim 1, wherein said soybean plant seed is from cultivar S36-B6 and is infected with *Fusarium virguliforme.*

5. Use of the compound of formula (I) 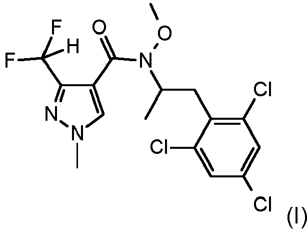 as a seed treatment for soybean seed, in order to suppress, control or reduce Soybean Sudden Death Syndrome in soybean plants grown from said soybean seed.

6. Use according to claim 5, wherein said soybean seed is from soybean cultivar S36-B6, and the Soybean Sudden Death syndrome is caused by *Fusarium virguilforme.*

## Patentansprüche

1. Verfahren zum Verringern des Auftretens von phytopathogenen Pilzen aus der Gruppe bestehend aus: *Fusarium virguliforme, Fusarium brasiliense* sp. nov., *Fusarium cuneirostrum* sp. nov. und *Fusarium tucumaniae* in Sojabohnenpflanzen, umfassend das Ausbringen einer wirksamen Menge einer Zusammensetzung, umfassend die Verbindung der Formel (I) 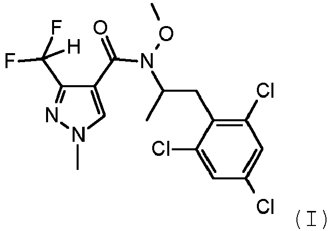 oder ein agrochemisch unbedenkliches Salz, Isomer, Stereoisomer, Diastereoisomer, Enantiomer oder Tautomer davon,
auf einen Sojabohnenpflanzensamen vor dem Pflanzen, wobei der Sojabohnenpflanzensamen mit einem Pilzstamm aus der Gruppe bestehend aus *Fusarium virguliforme, Fusarium brasiliense* sp. nov., *Fusarium cuneirostrum* sp. nov. und *Fusarium tucumaniae* infiziert ist.

2. Verfahren zum Unterdrücken, Bekämpfen oder Verringern von Sojabohnen-Sudden-Death-Syndrom in Sojabohnenpflanzen, umfassend das Pflanzen eines mit der Verbindung der Formel (I) gemäß Anspruch 1 behandelten Sojabohnenpflanzensamen im Boden, wobei der Sojabohnenpflanzensamen einen Vorläufer von Sojabohnen-Sudden-Death-Syndrom enthält, wobei der Vorläufer aus der Gruppe bestehend aus *Fusarium brasiliense* sp. nov., *Fusarium cuneirostrum* sp. nov. und *Fusarium tucumaniae* ausgewählt ist.

3. Verfahren zum Verringern der durch Sojabohnen-Sudden-Death-Syndrom verursachten Schädigung einer Sojabohnenpflanze oder eines Sojabohnenpflanzensamens, umfassend das Ausbringen der Verbindung der Formel (I) gemäß Anspruch 1 auf einen Sojabohnenpflanzensamen vor dem Pflanzen, wobei der Sojabohnenpflanzensamen einen Vorläufer von Sudden-Death-Syndrom enthält, wobei der Vorläufer aus der Gruppe bestehend aus *Fusarium brasiliense* sp. nov., *Fusarium cuneirostrum* sp. nov. und *Fusarium tucumaniae* ausgewählt ist; und das Pflanzen des Sojabohnenpflanzensamens.

4. Verfahren nach Anspruch 1, wobei der Sojabohnenpflanzensamen aus der Sorte S36-B6 stammt und mit *Fusarium virguliforme* infiziert ist.

5. Verwendung der Verbindung der Formel (I) 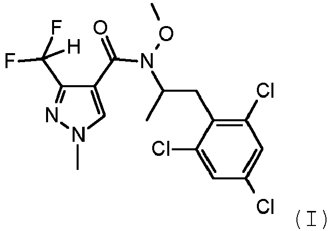 als Samenbehandlung für Sojabohnensamen zur Unterdrückung, Bekämpfung oder Verringerung von Sojabohnen-Sudden-Death-Syndrom in Sojabohnenpflanzen, die aus dem Sojabohnensamen gezogen werden.

6. Verwendung nach Anspruch 5, wobei der Sojabohnensamen aus der Sojabohnensorte S36-B6 stammt und das Sojabohnen-Sudden-Death-Syndrom durch *Fusarium virguliforme* verursacht wird.

## Revendications

1. Procédé pour la réduction de l'occurrence de champignons phytopathogènes choisis dans le groupe constitué par : *Fusarium virguliforme, Fusarium brasiliense* sp. nov., *Fusarium cuneirostrum* sp. nov. et *Fusarium tucumaniae,* dans des végétaux de soja, ledit procédé comprenant l'application d'une quantité efficace d'une composition comprenant le composé de formule (I) 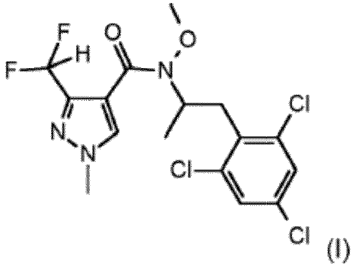 ou d'un sel, d'un isomère, d'un stéréoisomère, d'un diastéréoisomère, d'un énantiomère ou d'une forme tautomère acceptable sur le plan agrochimique correspondant(e),
à une semence de végétal de soja avant la plantation,
ladite semence de végétal de soja étant infectée par une souche fongique choisie dans le groupe constitué par *Fusarium virguliforme, Fusarium brasiliense* sp. nov., *Fusarium cuneirostrum* sp. nov. et *Fusarium tucumaniae.*

2. Procédé pour la suppression, la lutte contre ou la réduction du syndrome de la mort subite du soja dans des végétaux de soja comprenant la plantation dans le sol d'une semence de végétal de soja traitée avec le composé de formule (I) tel que défini dans la revendication 1, ladite semence de végétal de soja contenant un précurseur du syndrome de la mort subite du soja, ledit précurseur étant choisi dans le groupe constitué par *Fusarium brasiliense* sp. nov., *Fusarium cuneirostrum* sp. nov. et *Fusarium tucumaniae.*

3. Procédé pour la réduction de dommages à un végétal de soja ou une semence de végétal de soja causés par le syndrome de la mort subite du soja comprenant l'application à une semence de végétal de soja avant la plantation, du composé de formule (I) tel que défini dans la revendication 1, ladite semence de végétal de soja contenant un précurseur du syndrome de la mort subite choisi dans le groupe constitué par *Fusarium brasiliense* sp. nov., *Fusarium cuneirostrum* sp. nov. et *Fusarium tucumaniae* ; et la plantation de ladite semence de végétal de soja.

4. Procédé selon la revendication 1, ladite semence de végétal de soja provenant du cultivar S36-B6 et étant infectée par *Fusarium virguliforme.*

5. Utilisation du composé de formule (I) 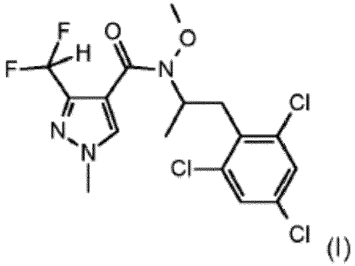 comme traitement de semence pour une semence de soja, afin de supprimer, de lutter contre ou de réduire le syndrome de la mort subite du soja dans des végétaux de soja qui ont poussé à partir de ladite semence de soja.

6. Utilisation selon la revendication 5, ladite semence de soja provenant du cultivar de soja S36-B6, et le syndrome de la mort subite du soja étant causé par *Fusarium virguliforme.*
